Europäisches Patentamt

European Patent Office

Office européen des brevets

(19)

(11) **EP 0 583 177 B1**

(12) **FASCICULE DE BREVET EUROPEEN**

(45) Date de publication et mention
de la délivrance du brevet:
**22.01.1997 Bulletin 1997/04**

(51) Int Cl.6: **C08F 2/32**, A61L 15/00

(21) Numéro de dépôt: **93401691.6**

(22) Date de dépôt: **30.06.1993**

(54) **Perfectionnement pour la préparation de poudre superabsorbante par polymérisation en suspension inverse de monomères acryliques**

Verbesserung in der Herstellung von superabsorbierendem Pulver durch Polymerisation von Acrylat-Monomeren in umgekehrte Suspension

Improvement in the preparation of superabsorbant powders by polymerisation of acrylic monomers in inverse emulsion

(84) Etats contractants désignés:
**AT BE CH DE DK ES FR GB GR IE IT LI LU NL PT SE**

(30) Priorité: **12.08.1992 FR 9209959**

(43) Date de publication de la demande:
**16.02.1994 Bulletin 1994/07**

(73) Titulaire: **ELF ATOCHEM S.A.**
**92800 Puteaux (FR)**

(72) Inventeurs:
• **Rebre, Shu Rong**
**F-94300 Vincennes (FR)**
• **Collette, Christian**
**F-75005 Paris (FR)**
• **Guerin, Thierry**
**F-94120 Fontenay S/Bois (FR)**

(74) Mandataire: **Haicour, Philippe**
**ELF ATOCHEM S.A.,**
**Département Propriété Industrielle.**
**4-8, Cours Michelet,**
**La Défense 10**
**92091 Paris Cédex 42 (FR)**

(56) Documents cités:
**EP-A- 0 441 507**        **US-A- 4 745 154**

## Description

La présente invention est relative à la production de poudres de polymères acryliques susceptibles d'absorber d'importantes quantités d'eau ou de fluides aqueux.

On sait réaliser des matériaux doués d'une forte capacité d'absorption d'eau par polymérisation en suspension inverse de monomères à insaturation éthylénique, plus particulièrement de monomères acryliques. Les poudres que l'on obtient ainsi gonflent fortement en présence d'eau, en donnant des gels à haute résistance mécanique. Ces propriétés sont mises à profit, entre autres, pour la fabrication d'articles sanitaires pour l'absorption et la rétention de liquides corporels.

Un perfectionnement important dans la production de telles poudres absorbantes est décrit dans le brevet européen EP 0441507. Il consiste à réaliser la polymérisation du monomère acrylique en au moins deux étapes. Dans une première étape, on réalise de façon classique une polymérisation en suspension inverse dans un solvant hydrocarboné, polymérisation qui conduit à la formation d'un gel. Dans une seconde étape, on fait absorber à ce gel une nouvelle charge de monomère, et on en provoque la polymérisation au sein même du gel précédemment formé. On peut, le cas échéant, répéter cette séquence d'absorption/polymérisation. On obtient de cette façon des résines polymères d'une granulométrie sensiblement plus forte que les résines de polymérisation en suspension inverse simple. Leur vitesse de gonflement en présence d'eau, le module élastique, la plasticité et la résistance au dégonflement sous pression du gel formé sont également sensiblement améliorés.

Mais pour atteindre ce résultat, il faut s'assurer que la seconde charge de monomère se polymérise bien au sein du premier gel formé et non à nouveau sous forme de suspension inverse dans la phase hydrocarbonée. Il est donc nécessaire de rendre le tensioactif, toujours présent dans le réacteur après la première étape de polymérisation, inapte à disperser la seconde charge de monomère. C'est la raison pour laquelle on doit procéder, selon l'art antérieur, au refroidissement de la masse résultant de la première polymérisation avant l'introduction de la seconde charge de monomère. Cette étape de refroidissement, qui est une caractéristique essentielle du procédé, est aussi extrêmement contraignante sur le plan industriel parce qu'elle allonge considérablement la durée de la fabrication et grève l'économie du procédé.

La présente invention constitue un perfectionnement important, des procédés d'obtention de poudres absorbantes par polymérisation radicalaire en suspension inverse en deux étapes, dans lequel l'absorption de la deuxième charge de monomère se fait à une température égale ou supérieure à 45°C, et qui consiste à opérer la mise en suspension inverse de la première charge de monomère à l'aide de certains tensioactifs polymérisables, également par polymérisation radicalaire, qui disparaîtront du milieu en tant qu'agent de mise en suspension au cours de la première étape de polymérisation.

Le choix de tensioactifs convenables de ce type est extrêmement délicat, notamment quant au résultat de la première polymérisation, et l'invention réside dans l'utilisation de composés répondant à la formule générale:

$$R_1-O-(CH_2-CH_2-O)_n-R_2$$

- où $R_1$ est une chaîne hydrocarbonée à au moins 9 atomes de carbone,
- où $R_2$ est un groupement polymérisable, reste acryloyle, méthacryloyle ou maléoyle,
- et où le degré de condensation en oxyde d'éthylène est compris entre 30 et 70.

Parmi les produits particulièrement intéressants appartenant à cette famille de tensioactifs polymérisables, on cite le monoester maléique du nonylphénol oxyéthyléné à 50 molécules d'oxyde d'éthylène, le monoester acrylique du nonylphénol oxyéthyléné à 45 molécules d'oxyde d'éthylène, le monoester méthacrylique du nonylphénol oxyéthyléné à 50 molécules d'oxyde d'éthylène.

D'une façon générale, les procédés de polymérisation en suspension inverse à deux étapes selon l'invention comportent les séquences suivantes qui sont détaillées dans les exemples:

a) préparation de la phase solvant,
b) préparation de la phase aqueuse de monomère (charge I),
c) mise en suspension du monomère et polymérisation I,
d) préparation de la phase aqueuse de monomère (charge II),
e) absorption de la charge II et polymérisation II,
f) séparation du polymère.

Il s'agit d'un processus opératoire qui pour l'essentiel est aujourd'hui bien connu de l'homme du métier et qui s'adresse à l'obtention de polymères de monomères insaturés hydrosolubles tels que l'acide acrylique, l'acide méthacrylique et leur amides méthylpropane-sulfoniques, ainsi que leurs dérivés non ioniques tels que l'acrylamide, le méthacrylamide et leur dérivés N,N-diméthyl substitués, le 2-hydroxyéthyl-acrylate ou méthacrylate; le N-méthylol-acrylamide ou méthacrylamide, ou encore leur dérivés azotés tels que le (diméthyl ou diéthyl)amino(éthyl ou propyl)-acrylate ou méthylacrylate et les sels d'ammonium quaternaires correspondants. L'acide acrylique est le monomère insaturé préféré. La polymérisation est initiée par les initiateurs de polymérisation radicalaire, qu'on préfère hydrosolubles puisqu'aussi bien le monomère que le polymère sont eux-mêmes hydrophiles; le persulfate de potassium étant particulièrement adapté au cas présent. Sont également bien connus de l'homme du métier, les divers auxiliaires utilisés pour l'obtention de polymères hydrophiles par polymérisation en suspension, parmi lesquels les colloïdes protecteurs qui accompagnent accessoirement les émulsifiants pour stabiliser physiquement des réactifs mis en suspension, par exemple les celluloses modifiées, les polyéthylènes ou leurs copolymères oxydés ou modifiés par l'anhydride maléique; parmi lesquels aussi des agents de réticulation pour la réticulation partielle des polymères hydrophiles produits, constitués de composés possédant au moins deux groupes insaturés copolymérisables avec le monomère insaturé (acide acrylique) dont le type est celui des di/triacrylates de polyols, ou susceptible de réagir avec ses produits de polymérisation, comme les diglycidyléthers de diols. Il est également connu que l'on peut utiliser comme solvant pour la mise en suspension inverse divers solvants hydrocarbonés aliphatiques, cycloaliphatiques ou aromatiques, le solvant ici préféré étant l'heptane.

Selon l'invention, le tensioactif polymérisable est introduit au cours de la séquence b, et comme il disparaît du milieu réactionnel en fin de séquence c, il n'est besoin de procéder qu'à un modeste refroidissement du réacteur, à une température égale ou supérieure à 45°C, pour que la séquence e) d'absorption du monomère se déroule à une température qui ne soit pas excessive, mais qui toutefois n'a pas besoin d'être inférieure à 35°C.

Il n'est pas impossible qu'après réalisation de cette séquence d'absorption, la suspension paraisse manquer de stabilité. On y remédie, si nécessaire, par addition d'un dispersant sur la composition duquel il n'y a pas de contrainte spéciale. On peut notamment utiliser comme émulsifiant de complément les émulsifiants connus de l'art antérieur, pour autant que les séquences d à f ne soient pas répétées.

La conduite du procédé se traduit par un gain inattendu et très substantiel de productivité dont on prendra la mesure en considérant que si le passage de la température d'absorption de la seconde charge de 18°C à 35°C, opération qui n'impose de refroidir le réacteur après la première polymérisation que vers 45°C, ne fait gagner en laboratoire qu'un temps en apparence négligeable, la productivité au stade industriel s'en trouve en revanche accrue de quelques 30 %.

Les exemples qui suivent feront mieux comprendre l'invention et en préciseront les conditions.

## EXEMPLE 1

### • *Séquence a*

Dans un réacteur d'un litre muni d'un dispositif d'introduction de réactifs solides ou liquides, d'un agitateur, d'un système de balayage par gaz neutre, d'une sonde de température et d'un dispositif de chauffage/réfrigération, on introduit 265,6 g d'heptane que l'on porte à 80°C et on y dissout sous agitation de 400 tours/minute, 0,92 g de polyéthylène modifié par l'anhydride maléique, un produit commercialisé par MITSUI PETROCHEMICAL INDUSTRIES Co sous le nom de Hi-Wax 1105A et qui servira de colloïde protecteur.

### • *Séquence b*

A part, on neutralise 92 g d'une solution aqueuse à 80 % en poids d'acide acrylique par 139,4 g de lessive de soude à 22 %. On ajoute 0,276 g d'hydroxyéthylcellulose, puis 5,5 g d'une solution aqueuse à 1 % de persulfate de potassium, 0,92 g d'une solution aqueuse à 2 % d'éthylène glycol diglycidyléther et 0,46 g de maléate ester de nonylphénol à 50 moles d'oxyde d'éthylène (Aérosol MEM-NP 50 de Cyanamid). A noter que cet émulsifiant est introduit dans la charge aqueuse, ce qui est assez inhabituel quand on veut provoquer la formation d'une suspension inverse.

### • *Séquence c*

Le réacteur étant maintenu sous agitation de 400 tours/minute et sous balayage d'azote à raison de 80 litres/minute, on y introduit peu à peu la phase aqueuse précédemment préparée qui passe en suspension inverse dans l'heptane. La température est portée à 70°C pour provoquer la polymérisation; elle est maintenue à ce niveau pendant 30 minutes. La température est alors ramenée à 45°C.

### • *Séquence d*

Pendant le déroulement de l'opération précédente, on procède à part à la neutralisation de 92 g de solution aqueuse à 80 % en poids d'acide acrylique par 139,4 g de lessive de soude à 22 %, puis on ajoute 5,5 g d'une solution aqueuse à 1 % de persulfate de potassium et 0,92 g d'une solution aqueuse à 2 % de éthylène glycol diglycidyléther. Cette phase aqueuse qui constitue la charge II de monomère est alors ramenée à 10°C.

### • *Séquence e*

L'agitation dans le réacteur est portée à 800 tours/minutes, le balayage d'azote étant maintenu à 80 litres/minutes. On y introduit peu à peu la charge II. La charge II étant introduite, on laisse l'absorption se poursuivre pendant 5 minutes à la température qui s'est fixée aux alentours de 35°C. Après quoi la température est portée à 70°C pour engager la seconde phase de polymérisation. On laisse la polymérisation se poursuivre pendant 30 minutes.

### • *Séquence finale f*

On élimine l'heptane et la majeure partie de l'eau par distillation. Après quoi on ajoute au contenu du réacteur 1,84 g d'une solution aqueuse à 2 % d'éthylène glycol diglycidyléther et on poursuit le séchage du produit sous azote.

On obtient ainsi une poudre avec une granulométrie moyenne de 370 µm avec un passant nul à 100 µm. Ce résultat n'est pas sensiblement modifié si la séquence d'absorption est conduite à 50°C.

## EXEMPLE 2 (CONTRE-EXEMPLE)

On répète les opérations de l'exemple 1, à la différence qu'à la séquence b, au lieu de 0,46 g d'Aérosol MEM-NP 50, on introduit comme émulsifiant 0,46 g de monoester acrylique de nonylphénol oxyéthyléné à 20 molécules d'oxyde d'éthylène.

La polymérisation se déroule de façon très anormale à la séquence c; il y a prise en masse du contenu du réacteur et il est impossible de poursuivre l'opération.

## EXEMPLE 3 (CONTRE-EXEMPLE)

Il s'agit d'un contre-exemple réalisé avec un émulsifiant selon l'art antérieur, qui fonctionne correctement lorsque l'on s'astreint à opérer la séquence d'absorption à basse température (20°C). On essaye sans succès de reproduire l'opération à la température d'absorption de 35°C.

### • Séquence a

Dans le dispositif décrit à l'exemple 1, on introduit 265,6 g d'heptane que l'on porte à 80°C et on y dissout sous agitation de 400 tours/minute, 0,74 g de di/tristéarate de saccharose et 0,92 g de polyéthylène modifié par l'anhydride maléique.

### • Séquence b

A part, on neutralise 92 g d'une solution aqueuse à 80 % en poids d'acide acrylique par 139,4 g de lessive de soude à 22 %. On ajoute 0,276 g d'hydroxyéthylcellulose, puis 5,5 g d'une solution aqueuse à 1 % de persulfate de potassium, et 0,92 g d'une solution aqueuse à 2 % d'éthylène glycol diglycidyléther.

Toutes les séquences suivantes c et d sont conduites comme dans l'exemple 1. Le produit final est une poudre dont la granulométrie moyenne de 150µm, et dont le passant à 100 µm est inacceptable avec une valeur supérieure à 15 %.

## Revendications

1. Procédé pour la réalisation de poudres superabsorbantes pour l'eau et les liquides aqueux par polymérisation de monomères insaturés hydrosolubles, comportant les opérations :

a) de préparation de la phase solvant hydrocarboné,
b) de préparation de la phase aqueuse de monomère (charge I),
c) de mise en suspension du monomère dans le solvant hydrocarboné et polymérisation I,
d) de préparation de la phase aqueuse de monomère (charge II),
e) d'absorption de la charge II et de polymérisation II,
f) de séparation du polymère,

caractérisé d'une part, en ce que le tensioactif utilisé pour la mise en suspension du monomère est un tensioactif polymérisable par polymérisation radicalaire et qui disparaît du milieu par polymérisation ou copolymérisation en fin d'opération c), tensioactif répondant à la formule générale $R_1$-O-$(CH_2$-$CH_2$-$O)_n$-$R_2$,

- où $R_1$ est une chaîne hydrocarbonée à au moins 9 atomes de carbone,
- où $R_2$ est un groupement polymérisable acryloyle, méthacryloyle ou maléoyle, et
- où le degré de condensation n en oxyde d'éthylène est compris entre 30 et 70,

procédé au cours duquel la température d'absorption de la charge II se déroule à une température égale ou supérieure à 45°C.

2. Procédé selon la revendication 1, caractérisé en ce que le tensioactif polymérisable est l'ester maléique de nonylphénol oxyéthyléné à 50 molécules d'oxyde d'éthylène, et qu'il est mis en solution dans la phase aqueuse au cours de la préparation de la phase aqueuse de monomère (charge I).

3. Procédé selon la revendication 1, caractérisé en ce que le polymère constituant la poudre superabsorbante est un polymère d'acide acrylique.

## Patentansprüche

1. Verfahren zur Herstellung superabsorbierender Pulver für Wasser und wäßrige Flüssigkeiten mittels Polymerisation wasserlöslicher ungesättigter Monomere, wobei dieses Verfahren die folgenden Verfahrensschritte umfaßt:

a) Herstellung der Kohlenwasserstofflösemittelphase;
b) Herstellung der wäßrigen Phase des Monomers (Charge I);
c) Suspendierung des Monomers in dem Kohlenwasserstofflösemittel und Polymerisation I;
d) Herstellung der wäßrigen Phase des Monomers (Charge II);

e) Absorption der Charge II und Polymerisation II;

f) Abtrennung des Polymers,

dadurch gekennzeichnet, daß das zur Suspendierung des Monomers verwendete Tensid ein durch radikalische Polymerisation polymerisierbares Tensid ist, das durch Polymerisation oder Copolymerisation am Ende des Verfahrensschrittes c) aus dem Milieu verschwindet, wobei das Tensid der allgemeinen Formel $R_1$-O-(CH$_2$-CH$_2$-O)$_n$-R$_2$ entspricht,

- in der $R_1$ eine Kohlenwasserstoffkette mit mindestens 9 Kohlenstoffatomen darstellt;
- in der $R_2$ eine polymerisierbare Acryloyl-, Methacryloyl-, oder Maleoylgruppe ist; und
- in der der Kondensationsgrad n an Ethylenoxid zwischen 30 und 70 beträgt,

wobei während des Verfahrens die Absorption der Charge II bei einer Temperatur von gleich oder höher als 45 °C abläuft.

2. Verfahren nach Anspruch 1, dadurch gekennzeichnet, daß das polymerisierbare Tensid ein Maleinsäurester des Oxyethylennonylphenols mit 50 Molekülen Ethylenoxid ist, der im Verlaufe der Herstellung der wäßrigen Phase des Monomers (Charge I) in der wäßrigen Phase in Lösung gebracht wird.

3. Verfahren nach Anspruch 1, dadurch gekennzeichnet, daß das Polymer, das das superabsorbierende Pulver darstellt, ein Acrylsäurepolymer ist.

**Claims**

1. Process for the production of powders which are superabsorbent for water and aqueous liquids, by polymerization of water-soluble unsaturated monomers, comprising the following operations:

    a) preparation of the hydrocarbon solvent phase,

    b) preparation of the aqueous monomer phase (charge I),

    c) bringing the monomer into suspension in the hydrocarbon solvent, and polymerization I,

    d) preparation of the aqueous monomer phase (charge II),

    e) absorption of charge II and polymerization II and

    f) isolation of the polymer,

characterized, on the one hand, in that the surfactant used for bringing the monomer into suspension is a surfactant which is polymerizable by radical polymerization and which disappears from the medium by polymerization or copolymerization at the end of operation c), surfactant corresponding to the general formula $R_1$-O-(CH$_2$-CH$_2$-O)$_n$-R$_2$,

- where $R_1$ is a hydrocarbon chain with at least 9 carbon atoms,
- where $R_2$ is a polymerizable acryloyl, methacryloyl or maleoyl group, and
- where the degree of condensation n in respect of ethylene oxide is between 30 and 70, during which process the temperature of absorption of the charge II proceeds at a temperature equal to or higher than 45°C.

2. Process according to Claim 1, characterized in that the polymerizable surfactant is the maleic acid ester of nonylphenol oxyethylenated with 50 molecules of ethylene oxide, and that it is dissolved in the aqueous phase during the preparation of the aqueous monomer phase (charge I).

3. Process according to Claim 1, characterized in that the polymer constituting the superabsorbent powder is an acrylic acid polymer.